# EUROPEAN PATENT APPLICATION

(11) **EP 3 812 023 A1**
(43) Date of publication of application: **28.04.2021**
(21) Application number: 19821532.9
(22) Date of filing: 21.06.2019
(51) Int. Cl.: B01D 11/02

(54) **SOLVENT COMPOSITION FOR NATURAL MATERIAL EXTRACTION**

(30) Priority: 21.06.2018 KR 20180071693
(71) Applicant: GS Caltex Corporation, Seoul 06141 (KR)
(72) Inventor: LEE, Julia, Daejeon 34048 (KR); KIM, Duk-Ki, Daejeon 34859 (KR)
(74) Representative: Zacco Sweden AB
(86) International application number: PCT/KR2019/007484
(87) International publication number: WO 2019/245317

(57) **Abstract**

The present invention relates to s solvent composition for natural material extraction, which is harmless to and safe for the human body, has excellent antibacterial activity, and can extract an effect substance from a natural material at improved yield. Containing 2,3-butanediol, the solvent composition for natural material extraction according to the present invention is harmless to and safe for the human body, has excellent antibacterial activity, and exhibits an improved yield in extracting an effective substance from a natural material. In addition, the solvent composition for natural material extraction contains 2,3-butanediol produced from biomass and thus does not cause the emission of greenhouse gases (CO₂).

## Description

### Technical Field

Disclosed herein is a solvent composition for extracting a natural material, which has no harmful effect on the human body, has an excellent antibacterial property and ensures improvement in yields of effective substances extracted from natural materials.

### Background Art

The food, cosmetics, pesticide and pharmaceutical and medical industries extract an effective substance from materials such as plants, marine algae, microalgae and the like in the natural system. The effective substance extracted can be used for essential oils, pigments, drug substances, fragrances and the like. To extract an effective substance from a natural material, a hot water extraction method, a super critical fluid extraction method, a solvent extraction method can be used. Chemical solvents, used to extract an effective substance from a natural material, include propylene glycol, 2-methyl-1,3-propanediol, butylene glycol, di-propylene glycol, glycerin, ethanol and the like.

The chemical solvent should not have a harmful effect on the human body because the chemical solvent directly contacts the human body in a state where the chemical solvent is melted in a natural substance. As a chemical solvent, 1,3-butylene glycol can irritate the skin.

As a chemical solvent, 1,3-propanediol can cause a reduction in yields of an effective substance extracted from a natural material while the component has no harmful effect on the human body.

As a chemical solvent, propylene glycol, butylene glycol, di-propylene glycol produced from a petrochemical material can emit more greenhouse gases (CO₂) than propylene glycol, butylene glycol, di-propylene glycol produced from a biomass material.

### Description of Invention

### Technical Problems

The present invention is directed to a solvent composition for extracting a natural material that may have no harmful effect on the human body, and may ensure improvement in yields of effective substances extracted from natural materials.

The present invention is also directed to a solvent composition for extracting a natural material that may have an excellent antibacterial property to prevent contamination and decay caused by microorganisms.

The present invention is also directed to a solvent composition for extracting a natural material that may not emit greenhouse gases (CO₂).

### Technical Solutions

A solvent composition for extracting a natural material according to the present invention may include 2,3-butanediol, such that the solvent composition for extracting a natural material has no harmful effect on the human body, has an excellent antibacterial property and ensures improvement in yields of effective substances extracted from natural materials.

A solvent composition for extracting a natural material according to the present invention may include 2,3-butanediol produced from biomass, such that the solvent composition for extracting a natural material emits no greenhouse gases (CO₂).

### Advantageous Effects

A solvent composition for extracting a natural material according to the present invention may include 2,3-butanediol, such that the solvent composition for extracting a natural material has no harmful effect on the human body and has an excellent antibacterial property. Thus, the solvent composition for extracting a natural material may be harmless to the human body even when the solvent composition for extracting a natural material is left in an extract.

Additionally, a solvent composition for extracting a natural material according to the present invention may include 2,3-butanediol, thereby improving yields of an effective substance extracted from a natural material.

Further, a solvent composition for extracting a natural material according to the present invention may include 2,3-butanediol produced from biomass, thereby reducing greenhouse gas (CO₂) emissions.

### Detailed Description of Exemplary Embodiments

The above-described aspects, features and advantages are specifically described hereunder such that one having ordinary skill in the art to which the present invention pertains can easily implement the technical spirit in the disclosure. In the disclosure, detailed description of known technologies in relation to the disclosure is omitted if it is deemed to make the gist of the disclosure unnecessarily vague. Below, preferred embodiments according to the disclosure are specifically described.

The inventive subject matter can be implemented in various different forms, and should not be construed as being limited only to the embodiments set forth herein. The embodiments are provided only as examples so that the disclosure will be thorough and complete, and will fully convey the scope of the inventive subject matter to those skilled in the art. Below, a solvent composition for extracting a natural material, which may have no harmful effect on the human body, have an excellent antibacterial property, and ensure a reduction in emissions of greenhouse gases (CO₂) and improvement in yields of effective substances extracted from natural materials, is specifically described.

### <Solvent composition for extracting natural material>

The solvent composition for extracting a natural material according to the present invention may include 2,3-butanediol.

2,3-butanediol, which is a sort of alcohol, may have four carbons and two hydroxyl groups (-OH). 2,3-butanediol may have a structure as in chemical formula 1 below, where the hydroxyl groups (-OH) are bonded to a second carbon and a third carbon.

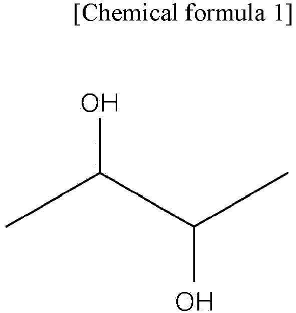

2,3-butanediol may be found in the natural system such as honey, raspberry and the like as well as in a wide range of fermented foods such as wine, cheese, vinegar and the like. Accordingly, 2,3-butanediol may be used for foods, cosmetics and the like.

2,3-butanediol according to the disclosure may include one or more of levo-2,3-butanediol and meso-2,3-butanediol. Levo-2,3-butanediol is characterized in that levo-2,3-butanediol may rotate a polarized surface counterclockwise when polarized light of 2,3-butanediol passes through a mirror image isomer of 2,3-butanediol. Meso-2,3-butanediol may be a compound where mirror-image symmetry is in a 2,3-butanediol molecule. In meso-2,3-butanediol, a stereocenter of the second carbon and the third carbon may be R, S or S, R, based on the Cahn-Ingold-Prelog (CIP) system.

Since 2,3-butanediol according to the disclosure includes one or more of levo-2,3-butanediol and meso-2,3-butanediol that have a specific conformation, 2,3-butanediol may ensure improvement in yields of an effective substance extracted from a natural material. Additionally, since 2,3-butanediol according to the disclosure includes one or more of levo-2,3-butanediol and meso-2,3-butanediol that have a specific conformation, 2,3-butanediol may ensure improvement in antibacterial properties.

Further, 2,3-butanediol according to the disclosure may include levo-2,3-butanediol and meso-2,3-butanediol, and a content ratio of levo-2,3-butanediol and meso-2,3-butanediol may be optimally adjusted for a target material (Target) to be extracted.

Depending on a target material (Target) to be extracted, as a greater levo-2,3-butanediol content may result in a greater yield of an effective substance extracted from a natural material, and a greater meso-2,3-butanediol content may result in may also result in a greater yield of an effective substance extracted from a natural material.

The natural material according to the disclosure may be a plant material. The natural material may be a natural material in the natural system and include a plant material, marine algae, microalgae and the like. The plant material, for example, may include green tea, red ginseng and the like. The solvent composition, which includes 2,3-butanediol according to the disclosure, may ensure the highest yield of an effective substance extracted from the plant material among the natural materials.

The solvent composition for extracting a natural material according to the present invention may include 2,3-butanediol produced from a petrochemical material or biomass. The solvent composition for extracting a natural material according to the disclosure may include 2,3-butanediol produced from biomass, for example. 2,3-butanediol produced from a petrochemical material may have excellent physical and chemical properties, but a large expense may be incurred because 2,3-butanediol produced from a petrochemical material needs to be separated and purified. Additionally, 2,3-butanediol produced from a petrochemical material may emit a large amount of greenhouse gases. Thus, the solvent composition for extracting a natural material according to the disclosure may preferably include 2,3-butanediol produced from biomass. Further, the solvent composition for extracting a natural material may be included in raw materials for cosmetics, agricultural products, or food.

Biomass may be an organic material produced from living things such as plants, microorganisms and the like. Generally, biomass may produce bioenergy such as methanol, ethanol, hydrogen and the like, based on thermal decomposition or fermentation of living things such as plants, microorganisms and the like. 2,3-butanediol according to the disclosure, produced from biomass based on fermentation, may ensure a reduction in entire greenhouse gas emissions with the help of photosynthesis-based carbon dioxide fixation.

2,3-butanediol which is produced from biomass in the fermentation process, and a liquid extract extracted from the natural material using 2,3-butanediol may be contained in raw material for cosmetics, agricultural products, food, pharmaceutical and medical products, fragrances or dyes.

2,3-butanediol which is produced from biomass in the fermentation process may not irritate the skin, and the component itself may be used as a raw material for cosmetics such as a moisturizer and the like and may be safe enough for intake. Additionally, 2,3-butanediol which is produced from biomass in the fermentation process is registered as a food additive in Korea. Accordingly, 2,3-butanediol itself may be used as a raw material for food. Further, 2,3-butanediol, which is produced from biomass in the fermentation process, may be used as a raw material for agricultural products because 2,3-butanediol facilitates growth of plants and prevents plant diseases. Thus, 2,3-butanediol, which is left after an effective substance is extracted from a natural material, may be included in and used as raw materials for cosmetics, agricultural products, food, pharmaceutical and medical products, fragrances or dyes, without being additionally separated.

2,3-butanediol, which is produced from biomass in the fermentation process, may be used to extract an effective substance from green tea.

For example, an extract extracted from green tea may include one or more of theobromine, catechin, epigallocatechin gallate (EGCG), epigallocatechin (EGC), epicatechin gallate (ECG), gallocatechin gallate (GCG) and gallocatechin (GC).

Additionally, the extract extracted from green tea may include amino acids, and, the amino acids, for example, may include one or more of L-theanine, alanine, arginine, asparagines, aspartic acid, cystine, γ-aminobutyric acid (GABA), glutamic acid, glycine, leucine, lysine, proline, serine, threonine and tryptophan.

Further, the extract extracted from green tea may include a pigment component, and the pigment component, for example, may include one or more of chlorophyll-a, pheophytin-a, and pheophytin-b.

In another example, 2,3-butanediol, which is produced from biomass in the fermentation process, may be used to extract an effective substance from red ginseng, and the extract extracted from red ginseng may include ginsenoside.

### <Preparing method of natural material extract>

A preparing method of a natural material extract according to the present invention may include mixing a solvent composition for extracting a natural material and a natural material; extracting by stirring or immersing the mixture of the solvent composition and the natural material at 20 to 80 °C for 1 to 24 hours; and removing solid materials from the extract extracted from the mixture and separating the extract.

The step of removing solid materials and separating the extract may be performed by filtering or centrifugation.

After the solid material is removed and the extract is separated, the extract may be additionally enriched by evaporation or distillation, or the solvent composition for extraction according to the present invention may be removed, to finally obtain an extract.

Without additional process of separating the solvent composition for extraction according to the present invention from the extract from which the solid material is removed, the solvent composition for extraction may be included in and used as raw materials for cosmetics, agricultural products, food, pharmaceutical and medical products, fragrances or dyes.

The solvent composition for extracting a natural material is the same as the solvent composition for extracting a natural material, described above, and the natural material is the same as the natural material, described above.

The mixture of the solvent composition for extracting a natural material according to the disclosure, and the natural material may be extracted by stirring at 20 to 80 °C for 1 to 24 hours. The extracted extract may include a compound or a composition as an effective substance. Preferably the mixture of the solvent composition for extracting a natural material according to the present invention, and the natural material may be extracted by stirring in a shaking incubator at 40 to 80 °C and 100 to 200 rpm for 1 to 24 hours.

When the solid material is removed from the extract based on filtering, a filter (e.g., polyvinylidene fluoride filter (PVDF) or a polytetrafluoroethylene (PTFE) filter having a pore diameter of 0.1 to 0.3 µm) may be used to filter the extract, and an extract may be finally obtained from the natural material.

Furthermore, the solvent composition for extracting a natural material according to the present invention may be mixed with a liquid extract extracted using a method different from the preparing method of a natural material extract according to the present invention, to be used as a preservative for preventing decay of an extract. Specifically, an effective component is extracted from a natural material using a solvent except 2,3-butanediol, and then the solvent composition for extracting a natural material including 2,3-butanediol according to the present invention may be used as a preservative for preventing the extract from decaying.

In other words, after a solvent including no 2,3-butanediol and a natural material are mixed and stirred to separate an extract, the extract may be mixed with the solvent composition for extracting a natural material according to the present invention to be prevented from decaying.

Below, aspects according to the present invention are described with reference to embodiments.

### <Embodiment>

### <Example 1>

2,3-btanediol (99.50 %) of GS Caltex, disclosed in Korean Patent No. 10-1581504, was used as a solvent composition for extracting a natural material. 2,3-butanediol was analyzed using the ASTM D6866 method, and a carbon content, which is produced by fermentation of the biomass, among the carbon content of 2,3-butanediol, was 100 %.

### <Example 2>

2,3-butanediol, where a content of levo-2,3-butanediol is 90 % or greater, was used as a solvent composition, among 2,3-butanediol (99.50%) in example 1.

### <Example 3>

2,3-butanediol where a content of meso-2,3-butanediol is 90 % or greater was used as a solvent composition, among 2,3-butanediol (99.50%) in example 1.

### <Example 4>

2,3-butanediol, where a content ratio of levo-2,3-butanediol and meso-2,3-butanediol is 1:1, was used as a solvent composition, among 2,3-butanediol (99.50%) in example 1.

### <Comparative example 1>

2,3-butanediol (98.00%) of ACROS Co. as a result of separation and purification of a petrochemical material was used as a solvent composition.

### <Comparative example 2>

1,3-propanediol (99.50%) of FERMANDIOL Co. was used as a solvent composition.

### <Comparative example 3>

1,3-butanediol (99.00%) of Daicel Co. was used as a solvent composition.

### <Comparative example 4>

1,3-propanediol (99.50%) of DuPont Tate & Lyle Bio Products Company, disclosed in US Patent No. 7759393 was used as a solvent composition.

### <Comparative example 5>

1,3-propanediol (98%) of Sigma-Aldrich was used as a solvent composition.

### <Comparative example 6>

Water (H₂O) was used as a solvent composition.

### <Comparative example 7>

An ethanol solution having concentration of 30% was used as a solvent composition.

### <Experimental example>

### 1. Evaluation of skin irritation

To evaluate skin irritation caused by solvent compositions for extracting a natural material in example 1 and comparative examples 1, 2 and 3, a patch test was conducted on a total of 32 people. The solvent compositions for extracting a natural material in example 1 and comparative examples 1, 2 and 3 were all at a concentration of 30 wt%, and the patch test was performed under the PCPC Safety Evaluation Guidelines and the ICDRG standard.

**Table 1**

| Skin irritation index under ICDRG standard | |
|---|---|
| Skin irritation index | Category |
| 0.00-0.25 | No skin irritation |
| 0.26-1.00 | Minor skin irritation |
| 1.01-2.50 | Meddle-level skin irritation |
| 2.51-4.00 | Severe skin irritation |

**Table 2**

| Evaluation of skin irritation | | | | |
|---|---|---|---|---|
| | Cases with skin irritation | Skin irritation index | | |
| | | 30 minutes | 24 hours | 48 hours |
| Example 1 | 0 | 0.0 | 0.0 | 0.0 |
| Comparative example 1 | 0 | 0.0 | 0.0 | 0.0 |
| Comparative example 2 | 0 | 0.0 | 0.0 | 0.0 |
| Comparative example 3 | 2 | 0.5 | 0.0 | 0.0 |

Table 2 above shows that the composition of 2,3-butanediol in example 1, the composition of 2,3-butanediol in comparative example 1, and the composition of 1,3-propanediol in comparative example 2 caused no skin irritation. However, the composition of 1,3-butanediol in comparative example 3 had a skin irritation index of 0.5. The composition of 1,3-butanediol caused minor skin irritation to two of the people in the patch test.

### 2. Evaluation of carbon dioxide emissions

To evaluate greenhouse gas emissions of the solvent compositions for extracting a natural material of example 1, comparative example 1, and comparative example 4, the non-anthropogenic CO₂ emission content, which is emitted from the solvent composition for extracting a natural material of example 1, comparative example 1, and comparative example 4 by decomposed into carbon dioxide and water by microorganism, was calculated.

**Table 3**

| Carbon dioxide emissions | | | | | | |
|---|---|---|---|---|---|---|
| | Molecular weight (g/mol) | Mass (kg) | Mol (mol) / 1 kg | CO₂ fixation (mol) | CO₂ emission (mol) | Total CO₂ emission to atmosphere (kg) |
| Example 1 | 90.12 | 1 | 11.10 | 44.40 | 44.40 | 0.00 |
| Comparative example 1 | 90.12 | 1 | 11.10 | 0.00 | 44.40 | 1.95 |
| Comparative example 4 | 76.094 | 1 | 13.10 | 39.40 | 39.40 | 0.00 |

Table 3 above shows that example 1, produced as a result of fermentation of biomass, achieved zero CO₂ emissions in the entire atmosphere due to reflecting the photosynthesis-based carbon dioxide fixation effect, unlike the 2,3-butanediol composition produced from a petrochemical material.

### 3. Relative content of green tea extract

To evaluate a relative content of a green tea extract, 0.2 g of dried green tea powder (by AMOREPACIFIC in South Korea) was mixed with 20 g of a solvent composition for extraction. Then the mixture was extracted by stirring in a shaking incubator of 60°C at 150 rpm for 6 hours. The solvent compositions in examples 2, 3 and 4 and comparative examples 5, 6 and 7 were used as the solvent composition for extraction. An extract of the dried green tea powder was filtered using a PVDF filter having a pore diameter of 0.2 µm. A relative content of the green tea extract according to the solvent composition for extraction was calculated based on the comparative example 7.

**Table 4**

| Relative content of green tea extract | | | | | | | |
|---|---|---|---|---|---|---|---|
| | Theobromine | Catechin | EGCG | EGC | ECG | GCG | GC |
| Example 2 | 1.80 | 0.54 | 0.84 | 1.17 | 0.97 | 0.52 | 0.52 |
| Example 3 | 1.23 | 0.27 | 0.43 | 0.64 | 0.56 | 1.18 | 0.34 |
| Example 4 | 0.45 | 0.55 | 0.81 | 1.03 | 0.79 | 1.59 | 0.48 |
| Comparative example 5 | 1.14 | 0.48 | 0.73 | 1.02 | 0.90 | 0.47 | 0.15 |
| Comparative example 6 | 1.46 | 1.30 | 0.16 | 0.80 | 0.32 | 1.00 | 1.04 |
| Comparative example 7 | 1.00 | 1.00 | 1.00 | 1.00 | 1.00 | 1.00 | 1.00 |

Table 4 above shows a relative LC-MS peak ratio of the green tea extract to the solvent composition for extraction of ethanol having the concentration of 30 % (comparative example 7). Table 4 above shows that example 2 in which levo-2,3-butanediol was used as a solvent composition for extracting a natural material had a higher relative content of the green tea extract than example 5 in which 1,3-propanediol was used as a solvent composition for extracting a natural material.

### 4. Theobromine, and Catechin content of green tea extract

In the experimental example 3, 0.2 g of dried green tea powder (by AMOREPACIFIC in South Korea) was extracted with Examples 2, 3, 4 and Comparative Examples 5, 6, 7 to calculate the actual quantitative results of Theobromine and Catechin.

**Table 5**

| Theobromine and Catechin content of green tea extract | | |
|---|---|---|
| | Theobromine(µg/mL) | Catechin(µg/mL) |
| Example 2 | 15.0 | 944.9 |
| Example 3 | 10.2 | 517.4 |
| Example 4 | 3.7 | 838.9 |
| Comparative example 5 | 9.4 | 830.0 |
| Comparative example 6 | 12.1 | 644.0 |
| Comparative example 7 | 8.3 | 811.8 |

Table 5 above shows that example 2 , in which levo-2,3-butanediol was used as a solvent composition for extracting a natural material had a higher theobromine and catechin content than comparative example 5 in which 1,3-propanediol was used as a solvent composition for extracting a natural material, comparative example 6 in which water was used as a solvent composition for extracting a natural material, and comparative example 7 in which 30 % of ethanol was used as solvent composition for extracting a natural material.

### 5. Amino acid content of green tea extract

In the experimental example 3, 0.2 g of dried green tea powder (by AMOREPACIFIC in South Korea) was extracted with Examples 2, 3, 4 and Comparative Examples 5, 6, 7 to calculate the actual quantitative results of amino acids.

**Table 6**

| Amino acid content of green tea extract | | | | | | |
|---|---|---|---|---|---|---|
| | Example 2 | Example 3 | Example 4 | Comparative example 5 | Comparative example 6 | Comparative example 7 |
| L-theanine (µmol/mL) | 1.92 | 1.43 | 1.89 | 1.72 | 2.37 | 2.21 |
| alanine (µmol/mL) | 0.06 | 0.03 | 0.05 | 0.04 | 0.05 | 0.05 |
| arginine (µmol/mL) | 0.08 | 0.03 | 0.06 | 0.19 | 0.57 | 0.51 |
| Asparagines (µmol/mL) | 0.04 | 0.03 | 0.04 | 0.03 | 0.05 | 0.05 |
| aspartic acid (µmol/mL) | 0.24 | 0.13 | 0.22 | 0.19 | 0.45 | 0.39 |
| cystine (µmol/mL) | Not detected | 0.03 | Not detected | Not detected | Not detected | Not detected |
| GABA (µmol/mL) | 0.04 | 0.03 | 0.04 | 0.04 | 0.04 | 0.05 |
| glutamic acid (µmol/mL) | 0.25 | 0.16 | 0.25 | 0.20 | 0.40 | 0.36 |
| glycine (µmol/mL) | 0.81 | 0.01 | 0.44 | 0.05 | 0.05 | 0.03 |
| Histidine (µmol/mL) | Not detected | Not detected | Not detected | 0.01 | 0.09 | 0.06 |
| Isoleucine (µmol/mL) | Not detected | Not detected | Not detected | Not detected | 0.01 | 0.01 |
| leucine (µmol/mL) | 0.01 | Not detected | 0.01 | 0.01 | 0.01 | 0.01 |
| lysine (µmol/mL) | 1.61 | 1.18 | 1.57 | 1.08 | 2.64 | 2.50 |
| proline (µmol/mL) | 0.03 | 0.02 | 0.03 | 0.02 | 0.04 | 0.04 |
| serine (µmol/mL) | 0.10 | 0.06 | 0.09 | 0.09 | 0.15 | 0.14 |
| Threonine (µmol/mL) | 0.03 | 0.02 | 0.03 | 0.03 | 0.04 | 0.04 |
| Tryptophan (µmol/mL) | 0.01 | 0.01 | 0.01 | 0.01 | 0.01 | 0.01 |
| tyrosine (µmol/mL) | Not detected | Not detected | Not detected | Not detected | 0.01 | 0.01 |
| Total content (µmol/mL) | 3.30 | 1.73 | 2.85 | 2.01 | 4.64 | 4.26 |

Table 6 above shows that example 2 in which levo-2,3-butanediol was used as a solvent composition for extracting a natural material had a total amino acid content that was 64.2% higher than that of comparative example 5 in which 1,3-propanediol was used as a solvent composition for extracting a natural material. Additionally, example 4, which is used as a solvent composition for extracting a natural material having a 50 % content of levo-2,3-butanediol, had a total amino acid content that was 41.8% higher than that of comparative example 5, which is used 1,3-propanediol as a solvent composition for extracting a natural material.

### 6. Relative content of main pigment of green tea extract

In the experimental example 3, 0.2 g of dried green tea powder (by AMOREPACIFIC in South Korea) was extracted with Examples 2, 3, 4 and Comparative Examples 5, 6, 7 to calculate the actual quantitative results of main pigments. A relative content of the main pigments of green tea extract according to the solvent composition for extraction was calculated based on the example 2.

**Table 7**

| Relative content of main pigment of green tea extract | | | | | | |
|---|---|---|---|---|---|---|
| | Example 2 | Example 3 | Example 4 | Comparative example 5 | Comparative example 6 | Comparative example 7 |
| Chlorophyll-a | 1.00 | 0.06 | 0.00 | 0.00 | 0.00 | 0.00 |
| Pheophytin-a | 1.00 | 0.66 | 0.85 | 0.02 | 0.01 | 0.13 |
| Pheophytin-b | 1.00 | 0.63 | 0.72 | 0.02 | 0.02 | 0.44 |

Table 7 shows the relative LC-MS peak ratio of green tea extract. Table 7 above shows that example 2 in which levo-2,3-butanediol was used as a solvent composition for extracting a natural material could extract chlorophyll-a, pheophytin-a, and pheophytin-b more efficiently than comparative examples 5, 6 and 7. Chlorophyll-a was not detected in a substance extracted using the compositions in comparative examples 5, 6 and 7. Peaks of pheophytin-a extracted by the compositions in comparative examples 5, 6 and 7 were level of 1 to 13 %, compared to a peak of the composition in example 2. Peaks of pheophytin-b extracted by the compositions in comparative examples 5, 6 and 7 were level of 2 to 44%, compared to a peak of the composition in example 2.

### 7. Ginsenoside content of red ginseng extract

Herein, 0.5 g of red ginseng powder (named Hansoowi and made in South Korea) was mixed with 20 g of a solvent composition for extraction. Then the extracting was carried out by stirring the mixture in a shaking incubator at 150 rpm for 24 hours. Examples 2 and 3, and comparative examples 2, 3 and 7 were used as the solvent composition for extraction. An extract of the red ginseng powder was centrifuged at 4°C and 10,000 rpm for 20 minutes, and then filtered using a PTFE syringe filter having a pore diameter of 0.2 µm. A total ginsenoside content of the red ginseng extract based on the solvent composition for extraction was calculated as a result of sum of each content of 22 kinds of ginsenosides analyzed using a high-performance liquid chromatography (HPLC).

**Table 8**

| Total ginsenoside content of red ginseng extract | |
|---|---|
| | Total ginsenosides(mg/g) |
| Example 2 | 0.41 |
| Example 3 | 0.43 |
| Comparative example 2 | 0.38 |
| Comparative example 3 | 0.40 |
| Comparative example 7 | 0.42 |

In Table 8, a total ginsenoside contents of example 2 using levo-2,3-butanediol as a solvent composition for extracting a natural material and example 3 using meso-2,3-butanediol as a solvent composition for extracting a natural material showed the same level with that of comparative example 7 using ethanol as a solvent composition for extracting a natural material, and showed higher than those of comparative example 2 using 1,3-propanediol as a solvent composition for extracting a natural material and comparative example 3 using 1,3-butanediol as a solvent composition for extracting a natural material.

### 8. Evaluation of antibacterial property

To evaluate an antibacterial property of the solvent compositions for extracting a natural material in examples 2 and 3, and comparative examples 3 and 4, minimum inhibitory concentration (MIC; %) of a total of five microorganisms was measured. A lower minimum inhibitory concentration (%) may denote a more excellent antibacterial property.

**Table 9**

| | Sort of microorganism | | | | |
|---|---|---|---|---|---|
| | *P. aeruginose* (MIC%) | *E.coli* (MIC%) | *S.aureus* (MIC%) | *C.albicans* (MIC%) | *A.niger* (MIC%) |
| Example 2 | 10 | 15 | 25 | 12.5 | 12.5 |
| Example 3 | 6.25 | 12.5 | 25 | 12.5 | 12.5 |
| Comparative example 3 | 12.5 | 17.5 | 25 | 12.5 | 12.5 |
| Comparative example 4 | 12.5 | 22 | 25 | 25 | 25 |

Table 9 shows that case of using the solvent composition having 99.50 or greater % of levo-2,3-butanediol or meso-2,3-butanediol had a more excellent antibacterial property than the case of using 1,3-butanediol or 1,3-propanediol as solvent composition. Accordingly, if using levo-2,3-butanediol or meso-2,3-butanediol of a minimum inhibitory concentration (%) or as a solvent composition for extracting a natural material or a preservative of an extracted natural material, the solvent composition may prevent contamination and decay caused by microorganisms.

The experimental examples show that a solvent composition for extracting a natural material including 2,3-butanediol has no harmful effect on the human body and has excellent antibacterial properties. Additionally, the solvent composition for extracting a natural material including 2,3-butanediol has the effect of increasing yields of an effective substance extracted from a natural material.

In particular, a solvent composition for extracting a natural material including 2,3-butanediol produced through fermentation of biomass may not emit greenhouse gases, may not cause skin irritation, and may increase yields of an effective substance extracted from a natural material.

The embodiments are described above with reference to a number of illustrative embodiments thereof. However, the present invention is not intended to limit the embodiments set forth herein, and numerous other modifications and embodiments can be devised by one skilled in the art without departing from the technical spirit of the disclosure. Further, the effects and predictable effects based on the configurations in the disclosure are to be included within the range of the disclosure though not explicitly described in the description of the embodiments.

## Claims

1. A solvent composition for extracting a natural material, comprising: 2,3-butanediol.

2. The solvent composition of claim 1, wherein 2,3-butanediol includes one or more of levo-2,3-butanediol and meso-2,3-butanediol.

3. The solvent composition of claim 1, wherein the solvent composition for extracting a natural material includes 2,3-butanediol produced from biomass.

4. The solvent composition of claim 1, wherein the natural material is plant materials, marine algae or microalgae.

5. The solvent composition of claim 4, wherein the plant materials is green tea or red ginseng.

6. A preparing method of a natural material extract, comprising:
mixing a solvent composition for extracting a natural material of any one of claims 1 to 3 and a natural material;
extracting by stirring or immersing a mixture of the solvent composition and the natural material at 20 to 80 °C for 1 to 24 hours; and
removing a solid material from a extract extracted from the mixture.

7. The preparing method of claim 6, wherein the preparing method further includes enriching the extract after a solid material is removed from the extract or removing the solvent composition for extracting a natural material.

8. The preparing method of claim 6, wherein the natural material is plant material, marine algae, or microalgae.

9. The preparing method of claim 8, wherein the plant material is green tea or red ginseng.

10. The preparing method of claim 9, wherein an extract extracted from green tea includes one or more of theobromine, catechin, epigallocatechin gallate (EGCG), epigallocatechin (EGC), epicatechin gallate (ECG), gallocatechin gallate (GCG) and gallocatechin (GC).

11. The preparing method of claim 9, wherein the extract extracted from green tea includes amino acids.

12. The preparing method of claim 11, wherein amino acids include one or more of L-theanine, alanine, arginine, asparagines, aspartic acid, cystine, γ-aminobutyric acid (GABA), glutamic acid, glycine, leucine, lysine, proline, serine, threonine and tryptophan.

13. The preparing method of claim 9 wherein the extract extracted from green tea includes pigment components.

14. The preparing method of claim 13, wherein the pigment components include one or more of chlorophyll-a, pheophytin-a and pheophytin-b.

15. The preparing method of claim 9, wherein an extract extracted from red ginseng includes ginsenoside.

16. A composition, comprising: the solvent composition of claim 3, and a liquid extract extracted from a natural material using the solvent composition.

17. The composition of claim 16, wherein the composition is used as raw materials for cosmetics, raw materials for agricultural products, raw materials for food, raw materials for pharmaceutical and medical products, materials for fragrances or materials for dyes.

18. A preserving method of a natural material extract, comprising:
mixing and stirring a solvent including no 2,3-butanediol and a natural material, and separating an extract; and
mixing the extract with the composition of any one of claim 1 to 3 as a preservative, and preventing decay of the extract.
